(19) Europäisches Patentamt
European Patent Office
Office européen des brevets

(11) EP 0 383 724 B2

(12) NEUE EUROPÄISCHE PATENTSCHRIFT

(45) Veröffentlichungstag und Bekanntmachung des Hinweises auf die Entscheidung über den Einspruch:
**17.03.1999 Patentblatt 1999/11**

(45) Hinweis auf die Patenterteilung:
**01.06.1994 Patentblatt 1994/22**

(21) Anmeldenummer: **90810081.1**

(22) Anmeldetag: **06.02.1990**

(51) Int Cl.6: **C08F 22/06**, C07C 57/38, C02F 5/10

# (54) Polymerisation von Maleinsäureanhydrid

Polymerization of maleic-acid anhydride

Polymérisation d'anhydride d'acide maléique

(84) Benannte Vertragsstaaten:
**BE CH DE ES FR GB GR IT LI NL**

(30) Priorität: **14.02.1989 GB 8903330**
**24.11.1989 GB 8926586**

(43) Veröffentlichungstag der Anmeldung:
**22.08.1990 Patentblatt 1990/34**

(73) Patentinhaber: **FMC CORPORATION (UK) LIMITED**
**Manchester M17 1WT (GB)**

(72) Erfinder:
- **Rideout, Jan, Dr.**
  **Horwich, Bolton BL6 6LN (GB)**
- **MacQuarrie, Duncan James**
  **Cheadle, Stockport SK8 2HD (GB)**

(74) Vertreter: **VOSSIUS & PARTNER**
**Postfach 86 07 67**
**81634 München (DE)**

(56) Entgegenhaltungen:
**EP-A- 261 589**
**GB-A- 1 411 063**
**US-A- 3 320 216**
**US-A- 3 474 114**
**US-A- 3 810 834**
**US-A- 4 374 733**

EP 0 383 724 B2

## Beschreibung

Vorliegende Erfindung betrifft die Polymensation von Maleinsäureanhydrid und die Produkte aus dieser Polymerisation.

Es ist bekannt, dass Maleinsäureanhydrid zu Polymeren mit variierenden Molekulargewichten verarbeitet werden kann. Verschiedene Polymerisationsverfahren sind in der Literatur beschrieben worden. Die Anwendung dieser Polymere, wie auch anderer Polycarbonsäuren als Kesselstein verhütende Mittel in wässrigen Systemen ist wohlbekannt.

Verfahren zur Polymerisation die sich des Toluols oder Xylols als Lösungsmittel bedienen sind bekannt, benötigen aber generell grosse Mengen an Initiator, z.B. 15-30 %, und konzentrierte Monomerlösungen in Lösungsmittel, um genügende Ausbeuten an Polymeren zu erzielen. Die GB 1 411 063 beschreibt die Anwendung von Xylol mit einem ortho-Isomerengehalt von weniger als 99%. Die Molekulargewichtsverteilung der mit diesem Verfahren erhaltenen Produkte ist üblicherweise sehr gross.

US 3,474,114 beschreibt Maleinsäureanhydridtelomere, die - nach dem damaligen Kenntnisstand des Patentinhabers - wenigstens ein chemisch gebundenes Lösemittel-Molekül des Lösemittels, in dem die Telomerisation durchgeführt wird, im Telomer haben.

Es wurde nun überraschenderweise gefunden, dass wenn die Polymerisation mit verdünnten Maleinsäureanhydridlösungen in ortho-Xylol oder einem substituierten ortho-Xylol und mit niedrigen Initiatormengen ausgeführt wird, eine hohe Ausbeute des Produktes erhalten wird, wobei das Produkt eine niedrige Molekulargewichtsverteilung hat und eine verbesserte Aktivität als Kesselstein kontrollierendes Mittel gegenüber bekannten Polycarbonsäuren zur Kesselsteinbehandlung zeigt. Die Produkte enthalten neue Polycarbonsäureanhydride, die, falls gewünscht, isoliert werden können und die hydrolisiert werden können, um die entsprechende neue Polycarbonsäure oder deren wasserlöslichen Salze zu erhalten. Die neuen Materialien haben eine überraschend hohe Aktivität als Kesselstein kontrollierendes Mittel.

Dementsprechend beschreibt vorliegende Erfindung ein Polymaleinsäureanhydrid mit einem mittleren Molekulargewicht gemessen mit Gelpermeations Chromatographie (GPC) zwischen 450 bis 800 und einem Polymolekularitätsindex zwischen 1,0 bis 1,15.

Der Polymolekularitätsindex ist das Verhältnis $M_w/M_n$, wobei $M_w$ das Gewichtsmittel der Molekulargewichte und $M_n$ das Zahlenmittel des Molekulargewichtes ist.

Das Polymaleinsäureanhydrid nach vorliegender Erfindung kann durch Polymerisation von Maleinsäureanhydrid hergestellt werden, unter Anwendung eines Peroxidinitiators in einer Menge von nicht grösser als 10 Gew.-%, bezogen auf das Anhydrid, in einem Lösungsmittel, das überwiegend ein o-Xylol der Formel I

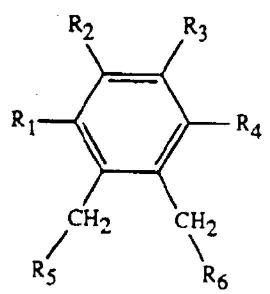

(I)

ist worin $R_1$, $R_2$, $R_3$ und $R_4$, unabhängig voneinander, ein Wasserstoffatom, eine $C_1$-$C_4$-Alkylgruppe oder eine Carboxylgruppe, $R_5$ und $R_6$, unabhängig voneinander, ein Wasserstoffatom oder eine Methylgruppe, oder $R_5$ und $R_6$ zusammen eine Methylen- oder Ethylengruppe bedeuten, und das Gewichtsverhältnis von Maleinsäureanhydrid zum Lösungsmittel nicht grösser als 1:3 und besonders bevorzugt nicht grösser als 1:4 ist.

Die Menge an Peroxid-Initiator kann von 1 bis 10 % Gew.-%, vorzugsweise von 4 bis 8 Gew.-%, bezogen auf das Maleinsäureanhydrid, betragen. Der Initiator kann aus den bekannten Peroxid-Initiatoren ausgewählt werden. Zum Beispiel kann es Dibenzoylperoxid, tert-Butylperbenzoat, Dicumylperoxid, tert.-Butylhydroperoxid oder, vorzugsweise, Di-tert.-butylperoxid.

Das Gewichtsverhältnis von Maleinsäureanhydrid zu Lösungsmittel kann von 1:3 zu 1:9, vorzugsweise von 1:3 bis 1:6 und insbesondere von 1:4 bis 1:6 sein. Dieses Gewichtsverhältnis kann zu Beginn der Polymerisationsreaktion auch höher sein, beispielsweise bis 1:2, vorausgesetzt Maleinsäureanhydrid wird während der Reaktion zuaddiert, so dass das endgültige Verhältnis nicht grösser als 1:3 ist.

Die Polymerisation, die stufenweise oder kontinuierlich ausgeführt werden kann, kann bei Temperaturen von

100 bis 200°C, vorzugsweise bei oder nahe der Rückflusstemperatur des Lösungsmittels, welche in der Praxis üblicherweise innerhalb des Bereiches von 120 bis 160°C und zum Beispiel bei 120 bis 146°C liegt. Die Reaktionszeit kann entsprechend der Reaktionstemperatur und dem zur Anwendung gelangenden Initiator variieren. Wird die Polymerisation beispielsweise in Gegenwart von Di-tert.-butylperoxid in o-Xylol bei Rückflusstemperatur ausgeführt, beträgt die Reaktionszeit üblicherweise zwischen 2 bis 5 Stunden.

Das Lösungsmittel enthält vorzugsweise mindestens 90 Gew.-% einer oder mehrerer Verbindungen der Formel I, besonders bevorzugt mehr als 95 Gew.-% und ganz besonders bevorzugt 97 Gew.-% solcher Verbindungen. Das Lösungsmittel kann kleinere Mengen anderer Xylol-Isomeren und Ethylbenzoyl enthalten.

Die oben beschriebene Polymerisation führt zu einem Polymaleinsäureanhydrid-Produkt, enthaltend neue Polycarbonsäureanhydride, üblicherweise mit anderen Polycarbonsäureanhydriden. Entsprechend beschreibt vorliegende Erfindung auch Polycarbonsäureanhydride der Formel II:

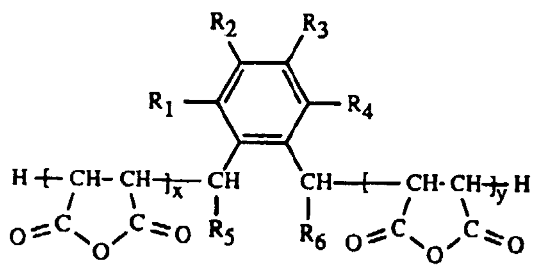

II

worin $R_1$, $R_2$, $R_3$, $R_4$, $R_5$ und $R_6$ die vorgenannte Bedeutung haben, x = 1, 2 oder 3 und y = 1, 2 oder 3 bedeuten, mit der Massgabe, dass x und y nicht gleichzeitig 1 sind.

Die Anhydride der Formel II werden üblicherweise als Resultat eines Polymerisationsverfahrens in Form von neuen Mischungen mit Polycarbonsäureanhydriden der Formel III erhalten:

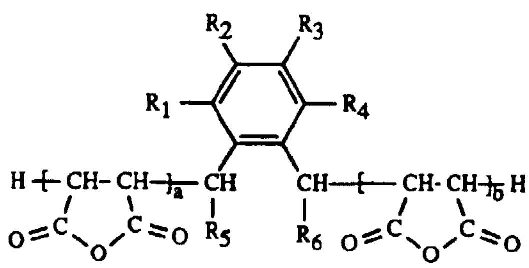

III

worin $R_1$, $R_2$, $R_3$, $R_4$, $R_5$ and $R_6$ die obengenannte Bedeutung haben, a und b 0 oder 1 bedeuten und die Summe von a + b 1 oder 2 bedeutet. Das Polymerisationsprodukt kann auch kleinere Mengen von nicht reagierten Verbindungen der Formel I enthalten.

Die Mischung enthält im allgemeinen mindestens 50 % des Anhydrides der Formel II. Wird das Polymerisationsprodukt mit Verfahren, welche die löslicheren Komponenten entfernen aufgearbeitet, wird der Prozentsatz von Anhydriden der Formel II in der Mischung erhöht.

In den Formeln I, II und III, wenn $R_1$, $R_2$, $R_3$ oder $R_4$ eine Alkylgruppe bedeutet, kann dies Methyl, Ethyl, n-Propyl, Isopropyl, n-Butyl, Isobutyl, sec.-Butyl, oder tert.-Butyl sein. Bevorzugte Verbindungen der Formel I, II und III umfassen solche, worin $R_1$ bis $R_6$ ein Wasserstoffatom bedeuten, solche, worin $R_1$ bis $R_3$, $R_5$ und $R_6$ ein Wasserstoffatom bedeuten und $R_4$ eine Methylgruppe bedeutet, solche, worin $R_1$ eine Methylgruppe und $R_2$ bis $R_6$ ein Wasserstoffatom bedeuten, solche, worin $R_1$, $R_4$, $R_5$ und $R_6$ ein Wasserstoffatom und $R_2$ und $R_3$ eine Methylgruppe bedeuten, solche worin $R_1$, $R_2$, $R_4$, $R_5$ und $R_6$ ein Wasserstoffatom und $R_3$ eine Methylgruppe bedeuten, solche, worin $R_1$, $R_3$, $R_4$, $R_5$ und $R_6$ ein Wasserstoffatom und $R_2$ eine Methylgruppe bedeuten, solche, worin $R_1$, $R_2$, $R_4$, $R_5$ und $R_6$ ein Wasserstoffatom und $R_3$ eine tert-Butylgruppe bedeuten, solche, worin $R_1$ und $R_3$ bis $R_6$ ein Wasserstoffatom und $R_2$ eine tert-Butylgruppe bedeuten, solche, worin $R_1$, $R_3$, $R_5$ und $R_6$ ein Wasserstoffatom und $R_2$ und $R_4$ eine Methylgruppe bedeuten, solche, worin $R_1$ und $R_3$ eine Methylgruppe und $R_2$, $R_4$, $R_5$ und $R_6$ ein Wasserstoffatom bedeuten, und solche, worin $R_1$, $R_4$, $R_5$ und $R_6$ ein Wasserstoffatom und $R_2$ und $R_3$ eine Carboxylgruppe bedeuten.

In bevorzugten Verbindungen der Formel II bedeutet x 1 und y 2. Die Verbindung der Formel III, die im Polymerisationsprodukt enthalten ist, besteht hauptsächlich aus Verbindungen, worin a 1 und b 1 ist.

In besonders bevorzugten Ausführungsformen der vorliegenden Erfindung bedeuten die Gruppen $R_1$ bis $R_6$ in den Formeln I, II und III ein Wasserstoffatom und in Formel II bedeutet x 1 und y 2. Dies in solchen Ausführungsformen worin die Verbindung der Formel I, beispielsweise die vorwiegende Verbindung im Polymerisations-Lösungsmittel, o-Xylol ist.

Gewöhnlich scheidet sich das Polymerisationsprodukt während der Polymerisationsreaktion ab. Es kann als das Anhydrid isoliert werden (üblicherweise eine Mischung von Anhydriden, wie vorbeschrieben, ebenso wie die einzelnen Verbindungen isoliert werden können, falls gewünscht, durch Reinigung) oder es kann zu den entsprechenden freien Polycarbonsäuren hydrolysiert werden, durch an sich übliche Methoden, zum Beispiel mittels Natriumhydroxid (gefolgt von einer Ansäuerung) oder Wasser. Die Hydrolyse kann am Schluss im Reaktionsgemisch oder am abgetrennten Polymer ausgeführt werden. Die Produkte können auch als wasserlösliche Salze, beispielsweise als Alkalimetallsalz, als alkalische Erdalkalimetallsalze oder als Ammoniumsalze isoliert werden.

Die Reinigung des Polymerisationsproduktes, um die einzelnen Verbindungen zu isolieren, kann durch konventionelle Methoden erfolgen, beispielsweise durch Umsetzung in ein Salz und mehrfache Rekristallisation des Salzes und Wiederherstellung der freien Säure, falls gewünscht, durch Ansäuerung einer wässrigen Lösung des Salzes, gefolgt durch Extraktion mit einem organischen Lösungsmittel.

Verbindungen der Formel II und Mischungen davon mit Verbindungen der Formel III können modiliziert werden, um andere Verbindungen der Formel II und Mischungen davon zu erhalten. Beispielsweise können Produkte, enthaltend eine Methylgruppe am aromatischen Ring durch an sich bekannte Verfahren oxidiert werden, um die Methylgruppe in eine Carboxylgruppe umzuwandeln.

Die Produkte nach vorliegender Erfindung sind wertvoll zur Inhibierung von Ablagerungen von Kesselstein bildenden Verbindungen aus Wasser oder wässrigen Systemen oder zur Modifizierung des Kristallisierverhaltens und Eigenschaften von ausgefällten Materialien.

Die erfindungsgemässen Produkte werden zweckmässig zum Wasser oder wässrigen System in Mengen von 0,1 bis 100 ppm, vorzugsweise von 0,5 zu 20 ppm, zugegeben.

Werden die erfindungsgemässen Produkte für die Inhibierung von Kesselsteinablagerungen und zur Ausfällung von Salzen aus wässrigen Lösungen angewendet, so sind die Produkte besonders wirksam zur Inhibierung von Kesselstein bildenden Ablagerungen von Salzen, abgeleitet von den Calcium-, Magnesium, Barium- oder Strontium-Kationen, und Anionen wie Sulphaten, Carbonaten, Hydroxiden, Phosphaten und Silikaten.

Im Bezug auf die wässrigen Systeme, die in Uebereinstimmung mit vorliegender Erfindung behandelt werden können, sind von besonderem Interesse Kühlwassersysteme, Dampferzeugersysteme, Meerwasserverdampfer, Ausrüstungen für die umgekehrte Osmose, Flaschenreinigungsanlagen, Papierbrei- und Papierherstellungsanlagen, Zukker-Verdampfungsanlagen, Bewässerungssysteme für Böden, hydrostatische Kocher, Gaswaschsysteme, Abgasentschwefelungsanlagen, geschlossene Heizsysteme, auf Wasser basierende Kühlsysteme, Oelherstellungs- und Bohrsysteme, Oelraffinerien, Kläranlagen, Kristallisatoren, Metallrückgewinnungssysteme und Photoentwicklungsbäder.

Die Produkte nach der Erfindung können allein angewendet werden oder in Verbindung mit anderen Materialien, die in der Behandlung von wässrigen Systemen bekannt sind.

In der Behandlung von Systemen, wie Kühlwassersystemen, Klimaanlagen, Dampferzeuger-Systemen, Meerwasserverdampfungs-Systemen, hydrostatischen Kochern und Heiz- und Kühlsystemen mit geschlossenem Kreislauf, können weitere Korrosionsinhibitoren verwendet werden, wie z.B. wasserlösliche Zinksalze, Phosphate, Polyphosphate, Phosphonsäuren und deren Salze, z.B. Hydroxyethyldiphosphonsäure (HEDP); Nitrilotrismethylenphosphonsäure und Methylaminodimethylenphosphoncarbonsäure und deren Salze, z.B. solche beschrieben in der Deutschen Offenlegungsschrift 26 32 774; 2-Hydroxyphosphonessigsäure; 2-Phosphonobutan-1,2,4-tricarbonsäure und jene, die in GB 1 572 406 beschrieben sind; Nitrate, z.B. Natriumnitrat; Nitrite, z.B. Natriumnitrit; Molybdate, z.B. Natriummbolybdat; Wolframate, Silikate, z.B. Natriumsilikate; Benztriazole; Bis-benztriazole oder Kupfer-desaktivierende Benztriazol- oder Tolutriazol-Derivate oder deren Derivate der Mannich-Basen; Mercaptobenztriazole; N-Acylsarcosine; N-Acyliminodiessigsäuren; Ethanolamine; Amine der Fettsäuren und Polycarbonsäuren, z.B. Polymaleinsäure und Polyacrylsäure, sowie deren entsprechenden Alkalimetallsalze, Copolymere des Maleinsäureanhydrides, z.B. Copolymere des Maleinsäureanhydrides und des sulfonierten Styrols, Copolymere der Acrylsäure, z.B. Copolymere der Acrylsäure und hydroxyalkylierten Acrylsäure, und substituierte Derivate der Polymaleinsäure und Polyacrylsäure und deren Copolymere. Darüberhinaus, in derartigen Systemen, kann das Produkt nach vorliegender Erfindung zusammen mit weiteren Dispergier- und/oder Fällungsverhinderungsmitteln (threshold agents) verwendet werden, z. B. polymerisierte Acrylsäure oder deren Salze, Phosphinopolycarbonsäuren (wie beschrieben und beansprucht in GB 1 458 235), den cotelomerischen Verbindungen beschrieben in EP 0 150 706, hydrolysierten Polyacrylnitrilen, polymerisierten Methacrylsäuren und deren Salzen, Polyacrylamiden und Copolymere davon mit Acryl- und Methacrylsäuren, Ligninsulfonsäuren und deren Salze, Tannin, Naphthalinsulfonsäure/Formaldehyd-Kondensationsprodukten, Stärke und deren Derivate, Cellulose, Acrylsäure/Niederalkylhydroxyacrylat-Copolymere, z.B. solche beschrieben in US 4 029 577, Styrol/Maleinsäureanhydrid-Copolymere und sulfonierte Styrolhornopolymere, z.B. solche beschrieben in

der US 4 374 733, und Kombinationen da von.

Spezifische Fällungsverhinderungsmittel, wie z.B. 2-Phosphonbutan-1,2,4- tricarbonsäure (PBSAM), Hydroxyethyldiphosphonsäure (HEDP), Alkylphosphonsäuren, Hydroxyphosphonessigsäure, 1-Aminoalkyl-1,1-diphosphonsäuren und deren Salze und Alkalimetallpolyphosphate können ebenfalls angewendet werden.

Besonders wichtige Addtiv-Zusammenstellungen sind solche, enthaltend die Produkte gemäss vorliegender Erfindung mit einer oder mehreren Polymaleinsäuren oder Copolymere davon, insbesondere Terpolymere mit Ethylacrylat und Vinylacetat, Polyacrylsäuren oder deren Copolymere oder substituierten Copolymere, 2-Hydroxyphosphonessigsäure, HEDP, PBSAM, Triazole, wie Tolutriazol, Molybdate und Nitrite.

Fällungsmittel, wie Alkalimetallorthophosphate, Carbonate, Sauerstoffänger(oxygen scavengers), wie Alkalimetallsulfite und Hydrazine, Maskierungsmittel (Sequestrierungsmittel), wie Nitrilotriessigsäure und deren Salze, Antischaummittel, wie Silikone, z.B. Polydimethylsiloxane, Distearylsebacinsäureamid, Distearyladipinsäureamid und verwandte Produkte, deriviert aus Ethylenoxid und/oder Propylenoxid-Kondensationen, in Addition zu Fettalkoholen, wie Caprylalkoholen und deren Ethylenoxid-Kondensate und Biocide, z.B. Amine, quaternäre Ammoniumverbindungen, Chlorophenole, Schwefelhaltige Verbindungen wie Sulphone, Methylenbisthiocyanate und -carbamate, Isothiazolone, bromierte Propionsäureamide, Triazine, Phosphoniumverbindungen, Chlor und Chlor-freisetzende Mittel, Brom und Brom-freisetzende Mittel, Ozon und organometallische Verbindungen, wie Tributylzinnoxide, können angewendet werden.

Die Erfindung ist durch nachfolgende Beispiele illustriert, in welchen die Prozentzahlen auf das Gewicht bezogen sind, ausser es wären andere Angeben gemacht, und in welchen die Molekulargewichtswerte $M_w$ und $M_n$ durch GPC erhalten werden. Die Massenspektrometer-Resultate (Fast atom bombardment mass spectrometry FAB-MS]) gemäss den Beispielen werden für die Polymaleinsäureanhydrodprodukte nach deren Umwandlung in die Säureform durch Auflösung in heissem Wasser erhalten. Die Gaschromatographie-Massenspektrometerresultate gemäss den Beispielen werden von der Trimethylsilylestern des Polymaleinsäureanhydrides erhalten.

<u>Beispiel 1:</u> 50 g Maleinsäureanhydrid und 290 g o-Xylol werden zum Rückfluss erhitzt, und 3 g Di-tert-butylperoxid werden innerhalb 15 Minuten zugetropft Nach 3.5 Stunden Reaktionszeit lässt man das Polymer sich von der Lösung abscheiden. Nach Kühlung auf Raumtemperatur wird die flüssige Phase abdekantiert. Man erhält 44 g polymeres Produkt (entsprechend einer Ausbeute von 88 % bezogen auf Maleinsäureanhydrid). Es ist keine weitere Reinigung erforderlich. Das erhaltene Polymer besitzt die mittleren Molekulargewichte $M_w$ = 550 und $M_n$ = 520 woraus sich $M_w/M_n$ = 1,058 ergibt. Die Gelpermeationschromatographie weist auf 4 Komponenten hin. Durch FAB-MS werden die beiden Hauptbestandteile als Verbindung der Formel II, worin $R_1$ bis $R_6$ Wasserstoff sind, x = 1 und y = 2 ist ([M + $H^+$ = 455), sowie als Verbindung der Formel II, worin $R_1$ bis $R_6$ Wasserstoff sind, x = 1 und y = 3 ist ($[M + H]^+$ = 571), identifiziert.

Eine Gaschromatographie-Massenspektrometrie der Trimethylsilylester identifiziert die restlichen beiden Komponenten als Verbindungen der Formel III, wobei im einen Fall $R_1$ bis $R_6$ Wasserstoff sind, a = 0 und b = 1 ist ($M^+$ = 366), und im anderen Fall $R_1$ bis $R_6$ Wasserstoff und a und b jeweils 1 sind ($M^+$ = 626).

<u>Beispiel 2:</u> Beispiel 1 wird wiederholt, mit der Ausnahme, dass 369 g o-Xylol eingesetzt werden. Die Produkteausbeute beträgt 42 g (84 %). Die mittleren Molekulargewichte des Produkts betragen $M_w$ = 500 und $M_n$ = 480. Das ergibt $M_w/M_n$ = 1,04.

<u>Beispiel 3:</u> Beispiel 1 wird wiederholt, mit der Ausnahme, dass 255 g o-Xylol eingesetzt werden. Die Produkteausbeute beträgt 47,3 g (94,6 %). Die mittleren Molekulargewichte des Produkts betragen $M_w$ = 490 und $M_n$ = 460. Das ergibt $M_w/M_n$ = 1,07.

<u>Beispiel 4:</u> Beispiel 1 wird wiederholt, mit der Ausnahme, dass 220 g o-Xylol eingesetzt werden. Die Produkteausbeute beträgt 47 g (94 %). Die mittleren Molekulargewichte des Produkts sind $M_w$ = 480 und $M_n$ = 440. Das ergibt $M_w/M_n$ = 1,09.

<u>Beispiel 5:</u> 149,6 g o-Xylol wird zum Rückfluss erhitzt, und 50,87 g Maleinsäureanhydrid werden innerhalb einer Stunde zugegeben. Im selben Zeitraum werden 3,6 g Di-tert-butylperoxid zugegeben. Nach 3,5 Stunden Kochen unter Rückfluss wird die entstandene Mischung auf 115°C abgekühlt. Nach Entfernung der flüssigen Phase erhält man 47,3 g des Polymerisationsprodukts, entsprechend einer Ausbeute von 93 % bezogen auf Maleinsäureanhydrid. Die mittleren Molekulargewichte des Produkts betragen $M_w$ = 590 und $M_n$ = 560, so dass $M_w/M_n$ = 1,05 ist.

<u>Beispiel 6:</u> 500 g o-Xylol werden zum Rückfluss erhitzt. Innerhalb eines Zeitraums von 2,5 Stunden werden 250 g Maleinsäureanhydrid und 30 g Di-tert-butylperoxid in separat Strömen zugegeben. Man lässt eine Stunde refluxieren und lässt die entstandene Mischung auf 115°-120°C abkühlen. Innerhalb der nächsten halben Stunde werden 440 ml Wasser zugetropft, und die Mischung noch weitere 15 Minuten bei 95°C gerührt. Durch Destillation wird das gesamte Xylol entfernt. Durch die Zugabe oder Entfernung von Wasser wird die Masse der Reaktionsmischung auf 880 g eingestellt. Unter Kühlung werden 382 g 46 %ige Natronlauge so zugegeben, dass die Reaktionstemperatur unter 60°C bleibt. Man erhält 1182 g einer 38,4 %igen Lösung des Natriumsalzes des Polymerisationsprodukts in Wasser. Nach Trocknung ergibt die Gelpermeationschromatographie des festen Produktes die Werte $M_w$ = 670, $M_n$ = 640 und folglich $M_w/M_n$ = 1,05.

Beispiel 7: 18,27 g Maleinsäureanhydrid und 50 g Durol (1,2,4,5-Tetramethylbenzol) werden auf 140°C erhitzt, und 1,1 g Di-tert-butylperoxid werden innerhalb von 5 Minuten zugegeben. Man belässt für weitere 3,5 Stunden bei 140°C, kühlt auf 100°C ab und dekantiert die flüssige Phase ab. Das verbleibende, feste Produkt wird in verdünnter Natronlauge gelöst, und die Lösung wird mit Toluol gewaschen, mit Salzsäure angesäuert und zur Trockene eingeengt. Das Polymerisationsprodukt (als Säure) wird vom entstandenen Kochsalz durch Behandlung mit Aceton und anschliessende Filtration abgetrennt. Nach Abziehen des Lösungsmittels erhält man das Polycarbonsäureprodukt in einer Ausbeute von 14 g (77 %). Die mittleren Molekulargewichte des Produktes betragen $M_w$= 660 und $M_n$ = 620. Das ergibt $M_w/M_n$= 1,06.

Die FAB-MS-Analyse zeigt peaks für eine Verbindung der Formel II, worin $R_1$, $R_4$, $R_5$ und $R_6$ Wasserstoff, $R_2$ und $R_3$ Methyl, x = 1 und y = 2 sind ($M + H^+ = 483,1$), sowie für eine Verbindung der Formel III, worin $R_1$, $R_4$, $R_5$ und $R_6$ Wasserstoff sind, $R_2$ und $R_3$ Methyl, a = 1 und b = 1 sind ($M + H^+ = 367,1$).

Beispiel 8: 15,27 g Maleinsäureanhydrid und 100 g 1,2,3-Trimethylbenzol werden auf 140°C erhitzt Innerhalb von 5 Minuten werden 0,92 g Di-tert-butylperoxid zugetropft. Die Reaktionsmischung wird drei Stunden lang bei 140°C belassen, auf 100°C abgekühlt und die flüssige Phase abdekantiert. Das Polymerisationsprodukt wird im Vakuum fünf Stunden lang bei 50°C getrocknet. Man erhält 8,98 (59 % bezogen auf Maleinsäureanhydrid). Die mittleren Molekulargewichte $M_w$ und $M_n$ betragen 646 und 613. Das ergibt $M_w/M_n$ = 1,05.

Die FAB-MS zeigt peaks für Verbindungen der Formel II, worin $R_1$ Methyl ist, $R_2$ bis $R_6$ Wasserstoff, x = 1 und y = 3 sind ($M + H^+ = 585,1$) und für die Verbindungen der Formel III, worin $R_1$ bis $R_3$ Wasserstoff, $R_4$ Methyl und $R_5$ und $R_6$ Wasserstoff a = 1 und b = 1 sind ($M + H^+ = 353,1$).

Beispiel 9: Unter den Bedingungen von Beispiel 8 wird statt 1,2,3-Trimethylbenzol 1,2,4-Trimethylbenzol eingesetzt. Die Produktionsausbeute beträgt 12,51 g (82 % bezogen auf Maleinsäureanhydrid). Die GPC Analyse ergibt die mittleren Molekulargewichte von $M_w$ = 638, $M_n$ = 616 und damit $M_w/M_n$ = 1,036.

FAB-MS zeigt peaks für Verbindungen der Formel II, worin $R_1$ und $R_3$ bis $R_6$ Wasserstoff sind und $R_2$ Methyl ist, bzw. sind $R_1$, $R_2$ und $R_4$ bis $R_6$ Wasserstoff und $R_3$ ist Methyl, in beiden Fällen ist x = 1 und y = 2 ($M + H^+ = 469,0$).

Beispiel 10: Unter den Bedingungen von Beispiel 7 werden statt der dort genannten Mischung 20 g Maleinsäureanhydrid, 80 g Isodurol (1,2,3,5-Tetramethylbenzol) und 1,2 Di-tert-butylperoxid eingesetzt Die Produkteausbeute beträgt 21,2 g (>100 % bezogen auf Maleinsäureanhydrid). Die mittleren Molekulargewichte des Produkts sind $M_w$ = 689 und $M_n$ = 657 und damit $M_w/M_n$ = 1,048.

Die FAB-MS ergibt peaks für eine Verbindung der Formel II, worin $R_1$ und $R_3$ Methyl, $R_2$ und $R_4$ bis $R_6$ Wasserstoff, x = 1 und y = 2 sind, sowie für eine Verbindung der Formel III, worin $R_1$, $R_3$, $R_5$ und $R_6$ Wasserstoff und a und b gleich eins sind. Die zugehörigen Muttedonen $M + H^+$ haben die Molekulargewichte 483,1 (Formel II) und 367,2 (Formel III).

Beispiel 11: Das Produkt des Beispiels 1 wird so in Wasser gelöst, dass sich eine Lösung von 20 Gew.% ergibt Man fügt p-Anisidin in wässrigem Aceton hinzu, bis der pH der Lösung 7 beträgt. Nach Trocknung wird der Rückstand zweimal aus Isopropanol umkristallisiert. Durch Auflösen des gereinigten Salzes in Natronlauge und Auswaschen des freien Amins mit Ether (sechsmal) kommt man wieder zur freien Säure. Behandlung der wässrigen Phase mit Salzsäure bis zu einem pH von eins und anschliessendes Einengen zur Trockene ergibt das gereinigte Produkt, vermischt mit Kochsalz. Durch Behandlung mit Aceton und Filtrieren wird das Kochsalz entfernt. Nach Abziehen des Lösungsmittels erhält man das gereinigte Produkt als gelben Farbstoff. Die GPC-Analyse des gereinigten Produkts zeigt nur einen peak, während die Mischung, die man bei Beispiel 1 erhält, entsprechend den vier massenspektrometrisch identifizierten Produkten 4 peaks ergibt. Das nach der Reinigung erhaltene Produkt entspricht einer Verbindung der Formel II, worin $R_1$ bis $R_6$ Wasserstoff, x gleich eins und y gleich zwei sind.

Beispiel 12: Unter den Bedingen von Beispiel 7 werden statt der dort genannten Mischung 3,7 g Maleinsäureanhydrid, 22 g 4-t-Butyl-o-xyld und 0,24 g Di-tert-butylperoxid eingesetzt Die Produktionsausbeute beträgt 3,3 g (89 % bezogen auf Maleinsäureanhydrid). Die mittleren Molekulargewichte des Produkts sind $M_w$ = 660, $M_n$ = 620 und damit $M_w/M_n$ = 1,07.

Vergleichsbeispiel

50 g Maleinsäureanhydrid werden in einem Gemisch von 99 % o-Xylol und 1 % m-Xylol gelöst und auf 130°C erhitzt. 14,89 g Di-tert-butylperoxid in 27,0 g desselben Lösungsmittelgemisches werden innerhalb von 15 Minuten zugetropft, und die Reaktions-Mischung wird 5 Stunden bei 130°C gehalten. Nach Kühlung auf 70°C und Entfernung der oberen Phase erhält man 60 g Produkt, was einer Ausbeute von 120 % entspräche. Die mittleren Molekulargewichte des Produkts sind $M_w$ = 890 und $M_n$ = 640. Damit ist $M_w/M_n$ = 1,39.

Test beispiele

Die Fähigkeit der Produkte, Ablagerungen von Erdalkalimetallsalzen zu verhindern, wird folgendermassen

gemessen:
Lösungen, welche die entsprechenden Kationen bzw. Anionen enthalten, werden zu einer Lösung vereinigt, aus der unter den im Folgenden genannten Testbedingungen ein Niederschlag ausfällt. Der das Erdalkalikation enthaltenden Lösung werden vor der Vermischung 2 ppm des jeweiligen Produkts zugefügt. Nach einer bestimmten Zeit wird die Konzentration des in der Lösung verbliebenen Kations gemessen, und der I %-Wert (Fällungsverhinderungswert) wird nach der folgenden Formel berechnet:

$$I\% = \frac{C_{end}-C_o}{C_{anf}-C_o} \cdot 100$$

Dabei bedeuten

$C_{end}$ : Konzentration des Kations bei Testende
$C_{anf}$ : Konzentration des Kations bei Test beginn
$C_o$ : Konzentration des Kations beim Ende eines Tests ohne Schwellenreagens

Reagentien mit I %-Werten über 50 % werden als geeignete Ablagerungsverhinderer angesehen.

Calciumcarbonat-Inhibierung

Bedingungen: $Ca^{2+}$: 150 mg/l, $Mg^{2+}$: 45 mg/l, $CO_3^{2-}$ 51 mg/l $HCO_3^-$ 269 mg/l, 70°C, 30 Minuten, Additivdosis 2 mg/l.

Die Ergebnisse sind in der folgenden Tabelle zusammengefasst:

| Additiv | % Inhibierung |
|---|---|
| Produkt aus Beispiel 1 | 93 |
| Produkt aus Beispiel 6 | 83 |
| Produkt aus Beispiel 7 | 82 |
| Produkt aus Beispiel 8 | 89 |
| Produkt aus Beispiel 9 | 88 |
| Produkt aus Beispiel 10 | 93 |
| Produkt aus Beispiel 11 | 95 |
| Produkt aus Beispiel 12 | 93 |
| Produkt aus Vergleichsbeispiel | 66 |
| Im Handel erhältliches Polyacrylat (Molekulargewicht 2000) | 67 |

Diese Ergebnisse zeigen die signifikante Ueberlegenheit der Produkte aus den Beispielen der vorliegenden Erfindung im Vergleich zum kommerziell erhältlichen Polyacrylat und zum Produkt des Vergleichsbeispiels, wenn die Eignung als Schwellenreagentien gemessen wird.

85°C Calciumcarbonat-Inhibierungs-Test bei vorhandenen Keimen

Mit diesem Test wird die Eignung von Additiven zur Verhinderung der Fällung von Calciumcarbonat unter Wachstumsbedingungen bei vorhandenen Keimen untersucht
Die Testlösung enthält 125 mg/l $Ca^{2+}$, 375 mg/l $Mg^{2+}$, 182 mg/l $CO_3^{2-}$ und 2 mg/l des Additivs. Jede Testlösung enthält ferner 0,02 g Calciumcarbonatkeime. Der Test dauert 30 Minuten bei 85°C, und die Konzentration der $Ca^{2+}$ wird sodann bestimmt. Die Ergebnisse, aus denen die Ueberlegenheit des Produkts aus Beispiel 1 klar hervorgeht, sind:

| Additiv | % Inhibierung |
|---|---|
| Produkt von Beispiel 1 | 69 |
| Produkt des Vergleichsbeispiels | 20 |
| Im Handel erhältliches Polyacrylat (Molekulargewicht 2000) | 12 |

Calciumsulfatfällungsverhinderung

In diesem Test untersucht man die Kesselsteinverhinderung bei sulfathartem Wasser. Die Testlösung enthält 2,94 g/l $Ca^{2+}$, 7,2 g/l $SO_4^{2-}$, 7,5 g/l NaCl bei einem pH von 8,0-8,5. Der Test wird über 24 Stunden geführt und zwar bei 70°C mit 5 mg/l des Additivs. Die erhaltenen Ergebnisse sind:

| Additiv | % Inhibierung 5 mg/l |
|---|---|
| Produkt aus Beispiel 1 | 92 |
| Im Handel erhältliches Polyacrylat, MG = 2000 | 31 |
| Im Handel erhältliches Polymaleinsäureanhydrid MG = 2000 | 12 |

Aus diesen Ergebnissen geht die wesentlich höhere Aktivität des Produktes aus Beispiel 1 klar hervor.

Bariumsulfatfällungsverhinderung

Bei diesem Test werden Seewasser und ein synthetisch gemischtes Wasser, das dem in geologischen Formationen nahe kommt, verwendet, um die Bedingungen in der Tiefe eines Bohrlochs zu simulieren. Die Testlösung enthält 136,3 mg/l $Ba^{2+}$, 335,1 mg/l $Sr^{2+}$, 1,666 g/l $Ca^{2+}$ and 1,38 $SO_4^{2-}$ und wird bis pH 5,5 gepuffert. Man testet 3 Stunden lang unter Schütteln im Wasserbad bei 70°C. Die Konzentration des $Ba^{2+}$ wird nach Testende bestimmt. Mit 20 mg/l des Produktes aus Beispiel 1 erzielt man so einen Fällungsverhinderungswert von 94 %.

Calciumcarbonatfällungsverhinderung

Bedingungen: 600 ppm $Ca^{2+}$, 300 ppm $Mg^{2+}$, 600 ppm $HCO_3^-$, 40°C, 24 Stunden unter Rühren bei 150 U/min.

Man erhält die folgenden Ergebnisse:

| Additiv | % Inhibierung |
|---|---|
| 15 ppm Produkt aus Beispiel 6 | 80 |
| 15 ppm Produkt aus Bcispiel 6 + 2,5 ppm Copolymer aus<br>6 mol Maleinsäureanhydrid<br>1 mol Ethylacetat<br>1 mol Vinylacetat | 85.2 |

**Patentansprüche**

**Patentansprüche für folgende Vertragsstaaten : BE, CH, DE, FR, GB, GR, IT, LI, NL**

1. Polymaleinsäureanhydrid mit einem durch Gelpermeations Chromatographie bestimmten Gewichtsmittel der Molekulargewichte zwischen 450 und 800 und einen Polymolekularitätsindex zwischen 1,0 und 1,15.

2. Das Produkt nach Anspruch 1 als freie Polysäure oder als deren wasserlösliches Polysalz.

3. Verfahren zur Herstellung von Polymaleinsäureanhydrid nach Anspruch 1, durch Polymerisation von Maleinsäureanhydrid unter Anwendung eines Peroxidinitiators, in einer Menge von nicht grösser als 10 Gew.%, bezogen auf das Maleinsäureanhydrid, in einem Lösungsmittel, das überwiegend ein o-Xylol der Formel I

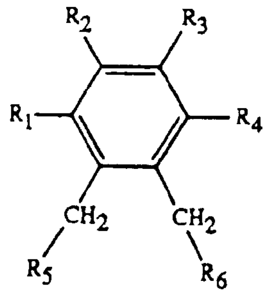

(I)

ist, worin $R_1$, $R_2$, $R_3$ und $R_4$, unabhängig voneinander, ein Wasserstoffatom, eine $C_1$-$C_4$-Alkylgruppe oder eine Carboxylgruppe, $R_5$ und $R_6$, unabhängig voneinander, ein Wasserstoffatom oder eine Methylgruppe, oder $R_5$ und $R_6$ zusammen eine Methylen- oder Ethylengruppe bedeuten und das Gewichtsverhältnis von Maleinsäureanhydrid zum Lösungsmittel nicht grösser als 1:3 ist.

4. Verfahren nach Anspruch 3, wobei das o-Xylol der Formel I ausgewählt ist aus der Reihe einer Verbindung, worin $R_1$ bis $R_6$ ein Wasserstoffatom bedeuten, einer Verbindung, worin $R_1$ bis $R_3$, $R_5$ und $R_6$ jeweils ein Wasserstoffatom und $R_4$ eine Methylgruppe bedeuten, einer Verbindung, worin $R_1$ eine Methylgruppe und $R_2$ bis $R_6$ jeweils ein Wasserstoffatom bedeuten, einer Verbindung, worin $R_1$, $R_4$, $R_5$ und $R_6$ jeweils ein Wasserstoffatom und $R_2$ und $R_3$ jeweils eine Methylgruppe bedeuten, einer Verbindung, worin $R_1$, $R_2$, $R_4$, $R_5$ und $R_6$ jeweils ein Wasserstoffatom und $R_3$ eine Methylgruppe bedeuten, einer Verbindung, worin $R_1$, $R_3$, $R_4$, $R_5$ und $R_6$ jeweils ein Wasserstoffatom und $R_2$ eine Methylgruppe bedeuten, einer Verbindung, worin $R_1$, $R_2$, $R_4$, $R_5$ und $R_6$ jeweils ein Wasserstoffatom und $R_3$ eine tert-Butylgruppe bedeuten, einer Verbindung, worin $R_1$ und $R_3$ bis $R_6$ ein Wasserstoffatom und $R_2$ eine tert-Butylgruppe bedeuten, einer Verbindung, worin $R_1$, $R_3$, $R_5$ und $R_6$ jeweils ein Wasserstoffatom und $R_2$ und $R_4$ jeweils eine Methylgruppe bedeuten, einer Verbindung, worin $R_1$ und $R_3$ jeweils eine Methylgruppe und $R_2$, $R_4$, $R_5$ und $R_6$ jeweils ein Wasserstoffatom bedeuten, einer Verbindung, worin $R_1$, $R_4$, $R_5$ und $R_6$ jeweils ein Wasserstoffatom und $R_2$ und $R_3$ jeweils eine Carboxylgruppe bedeuten, und Mischungen von zwei oder mehreren der genannten Verbindungen.

5. Verfahren nach Anspruch 3; bei dem das Lösungsmittel mehr als 95 Gew.-% einer oder mehrerer Verbindungen der Formel I enthält.

6. Verfahren nach Anspruch 5, bei dem das Lösungsmittel mehr als 97 Gew.-% einer oder mehrerer Verbindungen der Formel I enthält.

7. Verfahren nach Anspruch 3, bei dem das Gewichtsverhältins von Maleinsäureanhydrid zu Lösungsmittel nicht grösser als 1:4 ist

8. Verfahren nach Anspruch 3, bei dem das Gewichtsverhältins von Maleinsäureanhydrid zu Lösungsmittel von 1:3 bis 1:9 ist.

9. Verfahren nach Anspruch 8, bei dem das Gewichtsverhältins von Maleinsäureanhydrid zu Lösungsmittel von 1:3 bis 1:6 ist.

10. Verfahren nach Anspruch 3, bei dem die Menge des Peroxidinitiators 4 bis 8 Gew.%, bezogen auf Maleinsäureanhydrid, ist.

11. Verfahren nach Anspruch 3, bei dem der Peroxidinitiator Di-tert-butylperoxid ist.

12. Verfahren nach Anspruch 3, bei dem das erhaltene Anhydrid zur freien Säure oder deren wasserlöslichen Salze hydrolysiert wird.

13. Polycarbonsäureanhydrid der Formel II

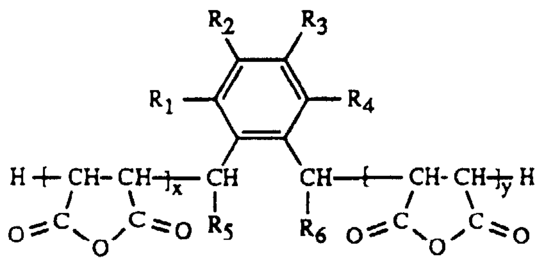

II

worin $R_1$, $R_2$, $R_3$ und $R_4$, unabhängig voneinander, ein Wasserstoffatom, eine $C_1$-$C_4$-Alkylgruppe oder eine Carboxylgruppe, $R_5$ und $R_6$, unabhängig voneinander, ein Wasserstoffatom oder eine Methylgruppe, oder $R_5$ und $R_6$ zusammen eine Methylen- oder Ethylengruppe bedeuten, x 1,2 oder 3 und y 1,2 oder 3 bedeuten, mit der Massgabe, dass x und y nicht zugleich 1 bedeuten.

**14.** Anhydrid nach Anspruch 13, worin $R_1$ bis $R_6$ jeweils ein Wasserstoffatom bedeuten oder $R_1$ bis $R_3$, $R_5$ und $R_6$ jeweils ein Wasserstoffatom und $R_4$ eine Methylgruppe bedeuten, oder $R_1$ eine Methylgruppe und $R_2$ bis $R_6$ jeweils ein Wasserstoffatom bedeuten, oder $R_1$, $R_4$, $R_5$ und $R_6$ jeweils ein Wasserstoffatom und $R_2$ und $R_3$ jeweils eine Methylgruppe bedeuten, oder $R_1$, $R_2$, $R_4$, $R_5$ und $R_6$ jeweils ein Wasserstoffatom und $R_3$ eine Methylgruppe bedeuten, oder $R_1$, $R_3$, $R_4$, $R_5$ und $R_6$ jeweils ein Wasserstoffatom und $R_2$ eine Methylgruppe bedeuten, oder $R_1$, $R_2$, $R_4$, $R_5$ und $R_6$ jeweils ein Wasserstoffatom und $R_3$ eine tert-Butylgruppe bedeuten, oder $R_1$ und $R_3$ bis $R_6$ jeweils ein Wasserstoffatom und $R_2$ eine tert-Butylgruppe bedeuten, oder $R_1$, $R_3$, $R_5$ und $R_6$ jeweils ein Wasserstoffatom und $R_2$ und $R_4$ jeweils eine Methylgruppe bedeuten, oder $R_1$ und $R_3$ jeweils eine Methylgruppe und $R_2$, $R_4$, $R_5$ und $R_6$ jeweils ein Wasserstoffatom bedeuten, oder $R_1$, $R_4$, $R_5$ und $R_6$ jeweils ein Wasserstoffatom und $R_2$ und $R_3$ jeweils eine Carboxylgruppe bedeuten.

**15.** Anhydrid nach Anspruch 14, wobei x 1 und y 2 sind.

**16.** Anhydrid nach Anspruch 14, wobei $R_1$ bis $R_6$ jeweils Wasserstoffatome sind.

**17.** Mischung enthaltend mindestens ein Anhydrid der Formel II nach Anspruch 13 und mindestens ein Anhydrid der Formel III

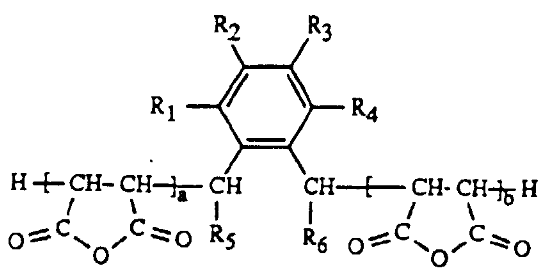

III

worin $R_1$, $R_2$, $R_3$, $R_4$, $R_5$ und $R_6$ die in Anspruch 13 angegebene Bedeutung haben, a und b jeweils 0 oder 1 sind und die Summe von a+b 1 oder 2 beträgt.

**18.** Mischung enthaltend mindestens ein Anhydrid der Formel II nach Anspruch 14 und mindestens ein Anhydrid der Formel III

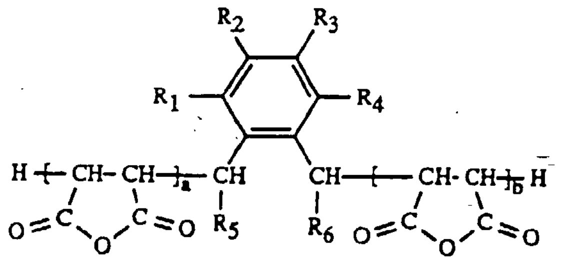

III

worin $R_1$, $R_2$, $R_3$, $R_4$, $R_5$ und $R_6$ die in Anspruch 14 angegebene Bedeutung haben, a und b jeweils 0 oder 1 bedeuten und die Summe von a + b 1 oder 2 beträgt

**19.** Mischung gemäss Anspruch 17, enthaltend mindestens ein Anhydrid der Formel II, worin x die Bedeutung von 1 und y die Bedeutung von 2 hat.

**20.** Polycarbonsäure oder deren wasserlösliches Salz, erhältlich durch Hydrolyse eines Anhydrides gemäss Anspruch 13.

**21.** Polycarbonsäure oder deren wasserlösliches Salz, erhältlich durch Hydrolyse der Mischung nach Anspruch 17.

**22.** Verfahren zur Inhibierung der Kesselsteinbildung aus Wasser oder einem wässrigen System, dadurch gekennzeichnet, dass dem Wasser oder den wässrigen Systemen ein Polymaleinsäureanhydrid nach Anspruch 1 zugefügt wird.

**23.** Verfahren zur Inhibierung der Kesselsteinbildung aus Wasser oder einem wässrigen Systemen, dadurch gekennzeichnet, dass dem Wasser oder den wässrigen Systemen ein Polycarbonsäureanhydrid nach Anspruch 13 oder eine Polycarbonsäure oder ein wasserlösliches Salz, erhältlich durch Hydrolyse des Anhydrides, zugefügt wird.

**Patentansprüche für folgenden Vertragsstaat : ES**

**1.** Verfahren zur Herstellung von Polymaleinsäureanhydrid, durch Polymerisation von Maleinsäureanhydrid unter Anwendung eines Peroxidinitiators, in einer Menge von nicht grösser als 10 Gew.%, bezogen auf das Maleinsäureanhydrid, in einem Lösungsmittel, das überwiegend ein o-Xylol der Formel I

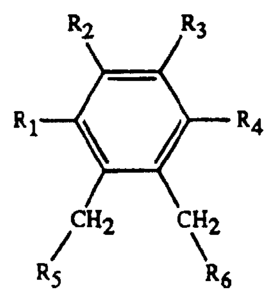

(I) :

ist, worin $R_1$, $R_2$, $R_3$ und $R_4$, unabhängig voneinander, ein Wasserstoffatom, eine $C_1$-$C_4$-Alkylgruppe oder eine Carboxylgruppe, $R_5$ und $R_6$, unabhängig voneinander, ein Wasserstoffatom oder eine Methylgruppe, oder $R_5$ und $R_6$ zusammen eine Methylen- oder Ethylengruppe bedeuten und das Gewichtsverhältuis von Maleinsäureanhydrid zum Lösungsmittel nicht grösser als 1:3 ist

**2.** Verfahren nach Anspruch 1 zur Herstellung von Polymaleinsäureanhydrid mit einem durch Gelpermeations Chromatographie bestimmten Gewichtsmittel der Molekulargewichte zwischen 450 und 800 und einen Polymolekularitätsindex zwischen 1,0 und 1,15.

3. Verfahren nach Anspruch 1 zur Herstellung des Produktes als freie Polysäure oder als deren wasserlösliches Polysalz.

4. Verfahren nach Anspruch 1, wobei das o-Xylol der Formel I ausgewählt ist aus der Reihe einer Verbindung, worin $R_1$ bis $R_6$ ein Wasserstoffatom bedeuten, einer Verbindung, worin $R_1$ bis $R_3$, $R_5$ und $R_6$ jeweils ein Wasserstoffatom und $R_4$ eine Methylgruppe bedeuten, einer Verbindung, worin $R_1$ eine Methylgruppe und $R_2$ bis $R_6$ jeweils ein Wasserstoffatom bedeuten, einer Verbindung, worin $R_1$, $R_4$, $R_5$ und $R_6$ jeweils ein Wasserstoffatom und $R_2$ und $R_3$ jeweils eine Methylgruppe bedeuten, einer Verbindung, worin $R_1$, $R_2$, $R_4$, $R_5$ und $R_6$ jeweils ein Wasserstoffatom und $R_3$ eine Methylgruppe bedeuten, einer Verbindung, worin $R_1$; $R_3$, $R_4$, $R_5$ und $R_6$ jeweils ein Wasserstoffatom und $R_2$ eine Methylgruppe bedeuten, einer Verbindung, worin $R_1$, $R_2$, $R_4$, $R_5$ und $R_6$ jeweils ein Wasserstoffatom und $R_3$ eine tert-Butylgruppe bedeuten, einer Verbindung, worin $R_1$ und $R_3$ bis $R_6$ ein Wasserstoffatom und $R_2$ eine tert-Butylgruppe bedeuten, einer Verbindung, worin $R_1$, $R_3$, $R_5$ und $R_6$ jeweils ein Wasserstoffatom und $R_2$ und $R_4$ jeweils eine Methylgruppe bedeuten, einer Verbindung, worin $R_1$ und $R_3$ jeweils eine Methylgruppe und $R_2$, $R_4$, $R_5$ und $R_6$ jeweils ein Wasserstoffatom bedeuten, einer Verbindung, worin $R_1$, $R_4$, $R_5$ und $R_6$ jeweils ein Wasserstoffatom und $R_2$ und $R_3$ jeweils eine Carboxylgruppe bedeuten, und Mischungen von zwei oder mehreren der genannten Verbindungen.

5. Verfahren nach Anspruch 1, bei dem das Lösungsmittel mehr als 95 Gew.-% einer oder mehrerer Verbindungen der Formel I enthält.

6. Verfahren nach Anspruch 5, bei dem das Lösungsmittel mehr als 97 Gew.-% einer oder mehrerer Verbindungen der Formel I enthält.

7. Verfahren nach Anspruch 1, bei dem das Gewichtsverhältins von Maleinsäureanhydrid zu Lösungsmittel nicht grösser als 1:4 ist.

8. Verfahren nach Anspruch 1, bei dem das Gewichtsverhältins von Maleinsäurelanhydrid zu Lösungsmittel von 1:3 bis 1:9 ist.

9. Verfahren nach Anspruch 8, bei dem das Gewichtsverhältins von Maleinsäureanhydrid zu Lösungsmittel von 1:3 bis 1:6 ist.

10. Verfahren nach Anspruch 1, bei dem die Menge des Peroxidinitiators 4 bis 8 Gew.%, bezogen auf Maleinsäureanhydrid, ist.

11. Verfahren nach Anspruch 1, bei dem der Peroxidinitiator Di-tert-butylperoxid ist.

12. Verfahren nach Anspruch 1, bei dem das erhaltene Anhydrid zur freien Säure oder deren wasserlöslichen Salze hydrolysiert wird.

13. Verfahren nach Anspruch 1 zur Herstellung von Polycarbonsäureanhydrid der Formel II

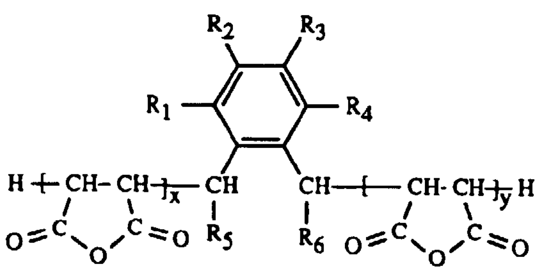

II

worin $R_1$, $R_2$, $R_3$ und $R_4$, unabhängig voneinander, ein Wasserstoffatom, eine $C_1$-$C_4$-Alkylgruppe oder eine Carboxylgruppe, $R_5$ und $R_6$, unabhängig voneinander, ein Wasserstoffatom oder eine Methylgruppe, oder $R_5$ und $R_6$ zusammen eine Methylen- oder Ethylengruppe bedeuten, x 1,2 oder 3 und y 1,2 oder 3 bedeuten, mit der Massgabe, dass x und y nicht zugleich 1 bedeuten.

**14.** Verfahren nach Anspruch 13 zur Herstellung von Anhydriden, worin $R_1$ bis $R_6$ jeweils ein Wasserstoffatom bedeuten oder $R_1$ bis $R_3$, $R_5$ und $R_6$ jeweils ein Wasserstoffatom und $R_4$ eine Methylgruppe bedeuten, oder $R_1$ eine Methylgruppe und $R_2$ bis $R_6$ jeweils ein Wasserstoffatom bedeuten, oder $R_1$, $R_4$, $R_5$ und $R_6$ jeweils ein Wasserstoffatom und $R_2$ und $R_3$ jeweils eine Methylgruppe bedeuten, oder $R_1$, $R_2$, $R_4$, $R_5$ und $R_6$ jeweils ein Wasserstoffatom und $R_3$ eine Methylgruppe bedeuten, oder $R_1$, $R_3$, $R_4$, $R_5$ und $R_6$ jeweils ein Wasserstoffatom und $R_2$ eine Methylgruppe bedeuten, oder $R_1$, $R_2$, $R_4$, $R_5$ und $R_6$ jeweils ein Wasserstoffatom und $R_3$ eine tert-Butylgruppe bedeuten, oder $R_1$ und $R_3$ bis $R_6$ jeweils ein Wasserstoffatom und $R_2$ eine tert-Butylgruppe bedeuten, oder $R_1$, $R_3$, $R_5$ und $R_6$ jeweils ein Wasserstoffatom und $R_2$ und $R_4$ jeweils eine Methylgruppe bedeuten, oder $R_1$ und $R_3$ jeweils eine Methylgruppe und $R_2$, $R_4$, $R_5$ und $R_6$ jeweils ein Wasserstoffatom bedeuten, oder $R_1$, $R_4$, $R_5$ und $R_6$ jeweils ein Wasserstoffatom und $R_2$ und $R_3$ jeweils eine Carboxylgruppe bedeuten.

**15.** Verfahren nach Anspruch 14 zur Herstellung von Anhydriden, wobei x 1 und y 2 sind.

**16.** Verfahren nach Anspruch 14 zur Herstellung von Anhydriden, wobei $R_1$ bis $R_6$ jeweils Wasserstoffatome sind.

**17.** Mischung enthaltend mindestens ein Anhydrid der Formel II nach Anspruch 13 und mindestens ein Anhydrid der Formel III

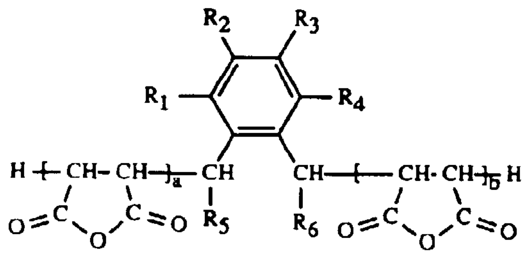

III

worin $R_1$, $R_2$, $R_3$, $R_4$, $R_5$ und $R_6$ die in Anspruch 13 angegebene Bedeutung haben, a und b jeweils 0 oder 1 sind und die Summe von a+b 1 oder 2 beträgt.

**18.** Mischung enthaltend mindestens ein Anhydrid der Formel II nach Anspruch 14 und mindestens ein Anhydrid der Formel III

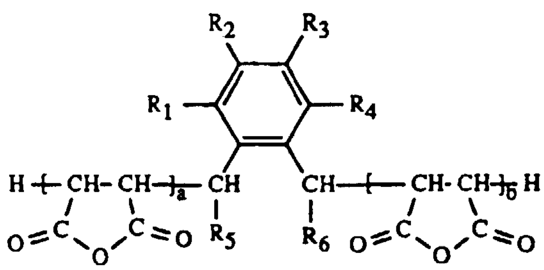

III

worin $R_1$, $R_2$, $R_3$, $R_4$, $R_5$ und $R_6$ die in Anspruch 14 angegebene Bedeutung haben, a und b jeweils 0 oder 1 bedeuten und die Summe von a + b 1 oder 2 beträgt.

**19.** Mischung gemäss Anspruch 17, enthaltend mindestens ein Anhydrid der Formel II, worin x die Bedeutung von 1 und y die Bedeutung von 2 hat.

**20.** Verfahren zur Herstellung von Polycarbonsäuren oder deren wasserlöslichen Salze, durch Hydrolyse eines Anhydrides gemäss Anspruch 13.

**21.** Verfahren zur Herstellung von Polycarbonsäuren oder deren wasserlöslichen Salze, durch Hydrolyse der Mischung nach Anspruch 17.

**22.** Verfahren zur Inhibierung der Kesselsteinbildung aus Wasser oder einem wässrigen System, dadurch gekennzeichnet, dass dem Wasser oder den wässrigen Systemen ein Polymaleinsäureanhydrid nach Anspruch 1 zugefügt wird.

**23.** Verfahren zur Inhibierung der Kesselsteinbildung aus Wasser oder einem wässrigen Systemen, dadurch gekennzeichnet, dass dem Wasser oder den wässrigen Systemen ein Polycarbonsäureanhydrid nach Anspruch 13 oder eine Polycarbonsäure oder ein wasserlösliches Salz, erhältlich durch Hydrolyse des Anhydrides, zugefügt wird.

## Claims

**Claims for the following Contracting States : BE, CH, DE, FR, GB, GR, IT, LI, NL**

**1.** A polymaleic acid anhydride having a weight average molecular weight determined by gel permeation chromatography of between 450 and 800 and a polydispersivity index of between 1.0 and 1.15.

**2.** The product according to claim 1 as a free poly acid or as a water-soluble poly salt thereof.

**3.** A method according to claim 1 for preparing polymaleic acid anhydride by polymerizing maleic acid anhydride using a peroxide intiator in an amount not greater than 10% by weight, based on the maleic acid anhydride, in a solvent which is predominantly an o-xylene of the formula I

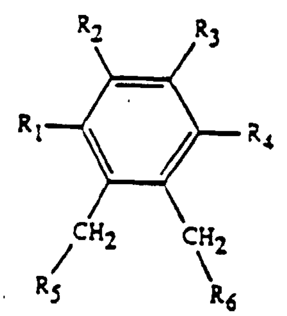

(I)

where $R_1$, $R_2$, $R_3$ and $R_4$ independently from each other denote a hydrogen atom, a $C_1$-$C_4$ alkyl group or a carboxyl group, $R_5$ and $R_6$ independently from each other denote a hydrogen atom or a methyl group, or $R_5$ and $R_6$ together denote a methylene or ethylene group and the weight ratio of maleic acid anhydride to the solvent is not greater than 1:3.

**4.** The method according to claim 3, wherein the o-xylene of the formula I is selected from a group consisting of a compound, in which $R_1$ to $R_6$ denote a hydrogen atom, a compound in which $R_1$ to $R_3$, $R_5$ and $R_6$ each denote a hydrogen atom and $R_4$ denotes a methyl group, a compound in which $R_1$ denotes a methyl group and $R_2$ to $R_6$ each denote a hydrogen atom, a compound in which $R_1$, $R_4$, $R_5$ and $R_6$ each denote a hydrogen atom and $R_2$ and $R_3$ each denote a methyl group, a compound, in which $R_1$, $R_2$, $R_4$, $R_5$ and $R_6$ each denote a hydrogen atom and $R_3$ denotes a methyl group, a compound in which $R_1$, $R_3$, $R_4$, $R_5$ and $R_6$ each denote a hydrogen atom and $R_2$ denotes a methyl group, a compound in which $R_1$, $R_2$, $R_4$, $R_5$ and $R_6$ each denote a hydrogen atom and $R_3$ denotes a tert.-butyl group, a compound in which $R_1$ and $R_3$ to $R_6$ denotes a hydrogen atom and $R_2$ denotes a tert.-butyl group, a compound in which $R_1$, $R_3$, $R_5$ and $R_6$ each denote a hydrogen atom and $R_2$ and $R_4$ each denote a methyl group, a compound in which $R_1$ and $R_3$ each denote a methyl group and $R_2$, $R_4$, $R_5$ and $R_6$ each denote a hydrogen atom, a compound in which $R_1$, $R_4$, $R_5$ and $R_6$ each denote a hydrogen atom and $R_2$ and $R_3$ each denote a carboxyl group, and mixtures of two or more of the afore-mentioned compounds.

**5.** The method according to claim 3, wherein the solvent contains more than 95% by weight of one or more compounds of formula I.

**6.** The method according to claim 5, wherein the solvent contains more than 97% by weight of one or more compounds

of formula I.

**7.** The method according to claim 3, wherein the weight ratio of maleic acid anhydride to solvent is not greater than 1:4.

**8.** The method according to claim 3, wherein the weight ratio of maleic acid anhydride to solvent ranges from 1:3 to 1:9.

**9.** The method according to claim 8, wherein the weight ratio of maleic acid anhydride to solvent ranges from 1:3 to 1:6.

**10.** The method according to claim 3, wherein the amount of peroxide initiator is 4 to 8% by weight, based on the maleic acid anhydride.

**11.** The method according to claim 3, wherein the peroxide initiator is di-tert.-butyl peroxide.

**12.** The method according to claim 3, wherein the resultant anhydride is hydrolyzed to the free acid or the water soluble salts thereof.

**13.** A polycarboxylic acid anhydride of formula II

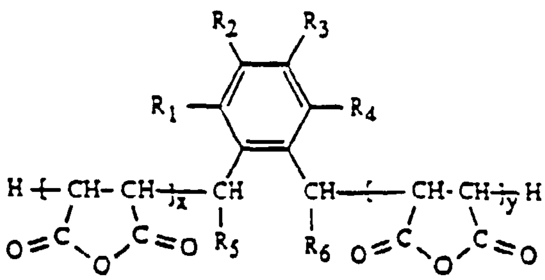

II

where $R_1$, $R_2$, $R_3$ and $R_4$ independently from each other denote a hydrogen atom, a $C_1$-$C_4$-alkyl group or a carboxyl group, $R_5$ and $R_6$ independently from each other denote a hydrogen atom or a methyl group, or $R_5$ and $R_6$ together denote a methylene or ethylene group, x denotes 1, 2 or 3 and y denotes 1, 2 or 3, with the proviso that x and y are not both 1.

**14.** An anhydride according to claim 13, where $R_1$ to $R_6$ each denote a hydrogen atom, or $R_1$ to $R_3$, $R_5$ and $R_6$ each denote a hydrogen atom and $R_4$ denotes a methyl group, or $R_1$ denotes a methyl group and $R_2$ to $R_6$ each denote a hydrogen atom, or $R_1$, $R_4$, $R_5$ and $R_6$ each denote a hydrogen atom and $R_2$ and $R_3$ each denote a methyl group, or $R_1$, $R_2$, $R_4$, $R_5$ and $R_6$ each denote a hydrogen atom and $R_3$ denotes a methyl group, or $R_1$, $R_3$, $R_4$, $R_5$ and $R_6$ each denote a hydrogen atom and $R_2$ denotes a methyl group, or $R_1$, $R_2$, $R_4$, $R_5$ and $R_6$ each denote a hydrogen atom and $R_3$ denotes a tert.-butyl group, or $R_1$ and $R_3$ to $R_6$ denotes a hydrogen atom and $R_2$ denotes a tert.-butyl group, or $R_1$, $R_3$, $R_5$ and $R_6$ each denote a hydrogen atom and $R_2$ and $R_4$ each denote a methyl group, or $R_1$ and $R_3$ each denote a methyl group and $R_2$, $R_4$, $R_5$ and $R_6$ each denote a hydrogen atom, or $R_1$, $R_4$, $R_5$ and $R_6$ each denote a hydrogen atom and $R_2$ and $R_3$ each denote a carboxyl group.

**15.** The anhydride according to claim 14, where x denotes 1 and y denotes 2.

**16.** The anhydride according to claim 14, where $R_1$ to $R_6$ each denote a hydrogen atom.

**17.** A mixture containing at least one anhydride of formula II according to claim 13 and at least one anhydride of formula III

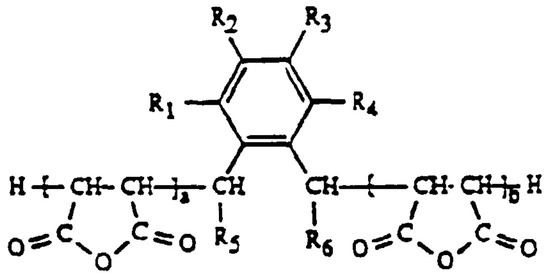

III

wherein $R_1$, $R_2$, $R_3$ $R_4$, $R_5$ and $R_6$ are as defined in claim 13, a and b each denote 0 or 1 and the sum of a+b is 1 or 2.

**18.** A mixture containing at least one anhydride of formula II according to claim 14 and at least one anhydride of formula III

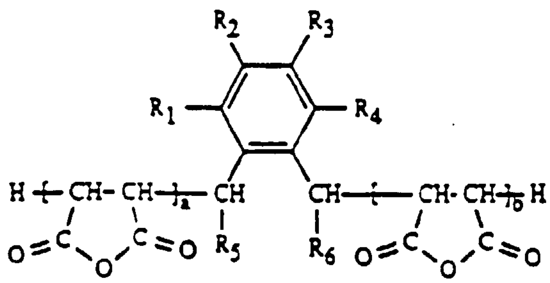

III

wherein $R_1$, $R_2$, $R_3$ $R_4$, $R_5$ and $R_6$ are as defined in claim 14, a and b each denote 0 or 1 and the sum of a+b is 1 or 2.

**19.** The mixture according to claim 17, containing at least one anhydride of formula II, where x denotes 1 and y denotes 2.

**20.** A polycarboxylic acid or water soluble salt thereof, obtainable by subjecting of an anhydride of claim 13 to hydrolysis.

**21.** A polycarboxylic acid or water soluble salt thereof, obtainable by subjecting the mixture of claim 17 to hydrolysis.

**22.** A method for inhibiting the formation of scale from water or an aqueous system characterized by the addition of a polymaleic acid anhydride according to claim 1 to the water or aqueous system.

**23.** A method for inhibiting the formation of scale from water or an aqueous system, characterized in that a polycarboxylic acid anhydride according to claim 13 or a polycarboxylic acid or a water soluble salt obtainable by the hydrolysis of the anhydride is added to the water or the aqueous system.

**Claims for the following Contracting State : ES**

**1.** A method for preparing polymaleic acid anhydride by polymerization of maleic acid anhydride using a peroxide initiator in an amount not greater than 10% by weight, based on the maleic acid anhydride in a solvent which is predominantly an o-xylene of formula I

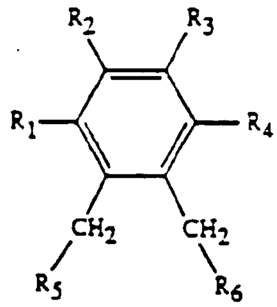

(I)

where $R_1$, $R_2$, $R_3$ and $R_4$ independently from each other denote a hydrogen atom, a $C_1$-$C_4$ alkyl group or a carboxyl group, $R_5$ and $R_6$ independently from each other denote a hydrogen atom or a methyl group, or $R_5$ and $R_6$ together denote a methylene or ethylene group and the weight ratio of maleic acid anhydride to the solvent is not greater than 1:3.

**2.** The method according to claim 1 for preparing a polymaleic acid anhydride having a weight average molecular weight determined by gel permeation chromatography of between 450 and 800 and a polydispersivity index of between 1.0 and 1.15.

**3.** The method according to claim 1 for preparing the product as a free polyacid or as a water soluble polysalt thereof.

**4.** The method according to claim 1, wherein the o-xylene of the formula I is selected from a group consisting of a compound, in which $R_1$ to $R_6$ denote a hydrogen atom, a compound in which $R_1$ to $R_3$, $R_5$ and $R_6$ each denote a hydrogen atom and $R_4$ denotes a methyl group, a compound in which $R_1$ denotes a methyl group and $R_2$ to $R_6$ each denote a hydrogen atom, a compound in which $R_1$, $R_4$, $R_5$ and $R_6$ each denote a hydrogen atom and $R_2$ and $R_3$ each denote a methyl group, a compound, in which $R_1$, $R_2$, $R_4$, $R_5$ and $R_6$ each denote a hydrogen atom and $R_3$ denotes a methyl group, a compound in which $R_1$, $R_3$, $R_4$, $R_5$ and $R_6$ each denote a hydrogen atom and $R_2$ denotes a methyl group, a compound in which $R_1$, $R_2$, $R_4$, $R_5$ and $R_6$ each denote a hydrogen atom and $R_3$ denotes a tert.-butyl group, a compound in which $R_1$ and $R_3$ to $R_6$ denotes a hydrogen atom and $R_2$ denotes a tert.-butyl group, a compound in which $R_1$, $R_3$, $R_5$ and $R_6$ each denote a hydrogen atom and $R_2$ and $R_4$ each denote a methyl group, a compound in which $R_1$ and $R_3$ each denote a methyl group and $R_2$, $R_4$, $R_5$ and $R_6$ each denote a hydrogen atom, a compound in which $R_1$, $R_4$, $R_5$ and $R_6$ each denote a hydrogen atom and $R_2$ and $R_3$ each denote a carboxyl group, and mixtures of two or more of the afore-mentioned compounds.

**5.** The method according to claim 1, wherein the solvent contains more than 95% by weight of one or more compounds of formula I.

**6.** The method according to claim 5, wherein the solvent contains more than 97% by weight of one or more compounds of formula I.

**7.** The method according to claim 1, wherein the weight ratio of maleic acid anhydride to solvent is not greater than 1:4.

**8.** The method according to claim 1, wherein the weight ratio of maleic acid anhydride to solvent ranges from 1:3 to 1:9.

**9.** The method according to claim 8, wherein the weight ratio of maleic acid annydride to solvent ranges from 1:3 to 1:6.

**10.** The method according to claim 1, wherein the amount of peroxide initiator is 4 to 8% by weight, based on the maleic acid anhydride.

**11.** The method according to claim 1, wherein the peroxide initiator is di-tert. -butyl peroxide.

**12.** The method according to claim 1, wherein the resultant anhydride is hydrolyzed to the free acid or the water soluble salts thereof.

**13.** The method according to claim 1 for preparing polycarboxylic acid anhydride of formula II

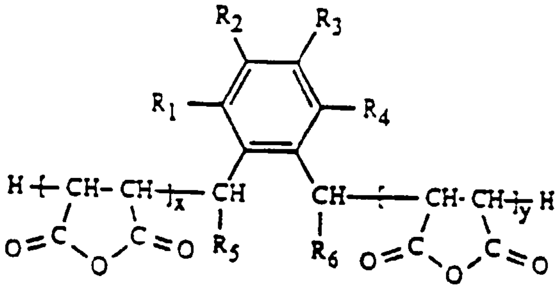

II

where $R_1$, $R_2$, $R_3$ and $R_4$ independently from each other denote a hydrogen atom, a $C_1$-$C_4$-alkyl group or a carboxyl group, $R_5$ and $R_6$ independently from each other denote a hydrogen atom or a methyl group, or $R_5$ and $R_6$ together denote a methylene or ethylene group, x denotes 1, 2 or 3 and y denotes 1, 2 or 3, with the proviso that x and y are not both 1.

**14.** The process according to claim 13 for preparing anhydrides, where $R_1$ to $R_6$ each denote a hydrogen atom, or $R_1$ to $R_3$, $R_5$ and $R_6$ each denote a hydrogen atom and $R_4$ denotes a methyl group, or R1 denotes a methyl group and $R_2$ to $R_6$ each denote a hydrogen atom, or $R_1$, $R_4$, $R_5$ and $R_6$ each denote a hydrogen atom and $R_2$ and $R_3$ each denote a methyl group, or $R_1$, $R_2$, $R_4$, $R_5$ and $R_6$ each denote a hydrogen atom and $R_3$ denotes a methyl group, or $R_1$, $R_3$, $R_4$, $R_5$ and $R_6$ each denote a hydrogen atom and $R_2$ denotes a methyl group, or $R_1$, $R_2$, $R_4$, $R_5$ and $R_6$ each denote a hydrogen atom and $R_3$ denotes a tert.-butyl group, or $R_1$ and $R_3$ to $R_6$ denotes a hydrogen atom and $R_2$ denotes a tert.-butyl group, or $R_1$, $R_3$, $R_5$ and $R_6$ each denote a hydrogen atom and $R_2$ and $R_4$ each denote a methyl group, or $R_1$ and $R_3$ each denote a methyl group and $R_2$, $R_4$, $R_5$ and $R_6$ each denote a hydrogen atom, or $R_1$, $R_4$, $R_5$ and $R_6$ each denote a hydrogen atom and $R_2$ and $R_3$ each denote a carboxyl group.

**15.** The method according to claim 14 for preparing anhydrides, where x denotes 1 and y denotes 2.

**16.** The method according to claim 14 for preparing anhydrides, where $R_1$ to $R_6$ each denote a hydrogen atom.

**17.** A mixture containing at least one anhydride of formula II according to claim 13 and at least one anhydride of formula III

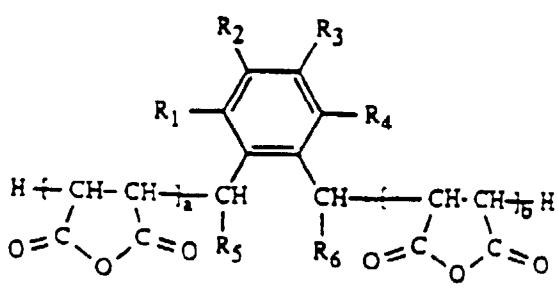

III

wherein $R_1$, $R_2$, $R_3$, $R_4$, $R_5$ and $R_6$ are as defined in claim 13, a and b each denote 0 or 1 and the sum of a+b is 1 or 2.

**18.** A mixture containing at least one anhydride of formula II according to claim 14 and at least one anhydride of formula III

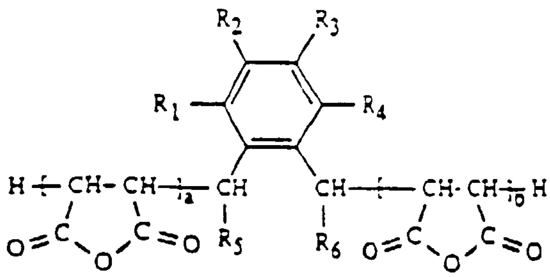

III

wherein $R_1$, $R_2$, $R_3$, $R_4$, $R_5$ and $R_6$ are as defined in claim 14, a and b each denote 0 or 1 and the sum of a+b is 1 or 2.

**19.** The mixture according to claim 17, containing at least one anhydride of formula II, where x denotes 1 and y denotes 2.

**20.** A method for preparing polycarboxylic acids or water soluble salts thereof, obtainable by subjecting of an anhydride of claim 13 to hydrolysis.

**21.** A method for preparing polycarboxylic acids or water soluble salts thereof, obtainable by subjecting the mixture of claim 17 to hydrolysis.

**22.** A method for inhibiting the formation of scale from water or an aqueous system characterized by the addition of a polymaleic acid anhydride according to claim 1 to the water or aqueous system.

**23.** A method for inhibiting the formation of scale from water or an aqueous system, characterized in that a polycarboxylic acid anhydride according to claim 13 or a polycarboxylic acid or a water soluble salt obtainable by the hydrolysis of the anhydride is added to the water or aqueous system.

**Revendications**

**Revendications pour les Etats contractants suivants : BE, CH, DE, FR, GB, GR, IT, LI, NL**

**1.** Poly(anhydride) d'acide maléique dont la moyenne pondérale des poids moléculaires déterminée au moyen de chromatographie par permeation de gel se situe entre 450 et 800 et dont l'indice de polymolécularité se situe entre 1,0 et 1,15.

**2.** Le produit selon la revendication 1 sous forme de polyacide libre ou de son polysel soluble dans l'eau.

**3.** Procédé selon la revendication 1 destiné à la fabrication de poly(anhydride) d'acide maléique par polymérisation d'anhydride d'acide maléique avec utilisation d'un initiateur de type peroxyde dans une quantité non supérieure à 10 % en poids rapportée à l'anhydride d'acide maléique dans un solvant qui est principalement un o-xylène de la formule I

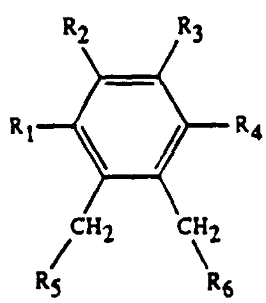

(I)

dans laquelle $R_1$, $R_2$, $R_3$ et $R_4$ signifient, indépendamment les uns des autres, un atome d'hydrogène, un groupe alkyle $C_1$-$C_4$ ou un groupe carboxyle, $R_5$ et $R_6$ signifient, indépendamment l'un de l'autre, un atome d'hydrogène ou un groupe méthyle, ou $R_5$ et $R_6$ ensemble signifient un groupe méthylène ou un groupe éthylène, et dans lequel le rapport des poids entre l'anhydride d'acide maléique et le solvant n'est pas supérieur à 1:3.

4. Procédé selon la revendication 3, dans lequel l'o-xylène de la formule I est choisi parmi la série d'un composé dans lequel $R_1$ à $R_6$ signifient un atome d'hydrogène, d'un composé dans lequel $R_1$ à $R_3$, $R_5$ et $R_6$ signifient respectivement un atome d'hydrogène et $R_4$ un groupe méthyle, d'un composé dans lequel $R_1$ signifie un groupe méthyle et $R_2$ à $R_6$ respectivement un atome d'hydrogène, d'un composé dans lequel $R_1$, $R_4$, $R_5$ et $R_6$ signifient respectivement un atome d'hydrogène et $R_2$ et $R_3$ respectivement un groupe méthyle, d'un composé dans lequel $R_1$, $R_2$, $R_4$, $R_5$ et $R_6$ signifient respectivement un atome d'hydrogène et $R_3$ un groupe méthyle, d'un composé dans lequel $R_1$, $R_3$, $R_4$, $R_5$ et $R_6$ signifient respectivement un atome d'hydrogène et $R_2$ un groupe méthyle, d'un composé dans lequel $R_1$, $R_2$, $R_4$, $R_5$ et $R_6$ signifient respectivement un atome d'hydrogène et $R_3$ un groupe tert-butyle, d'un composé dans lequel $R_1$ et $R_3$ à $R_6$ signifient un atome d'hydrogène et $R_2$ un groupe tert-butyle, d'un composé dans lequel $R_1$, $R_3$, $R_5$ et $R_6$ signifient respectivement un atome d'hydrogène et $R_2$ et $R_4$ respectivement un groupe méthyle, d'un composé dans lequel $R_1$ et $R_3$ signifient respectivement un groupe méthyle et $R_2$, $R_4$, $R_5$ et $R_6$ respectivement un atome d'hydrogène, d'un composé dans lequel $R_1$, $R_4$, $R_5$ et $R_6$ signifient respectivement un atome d'hydrogène et $R_2$ et $R_3$ respectivement un groupe carboxyle, et de mélanges de deux ou de plusieurs des composés cités.

5. Procédé selon la revendication 3, dans lequel le solvant contient plus de 95 % en poids d'un ou de plusieurs composés de la formule I.

6. Procédé selon la revendication 5, dans lequel le solvant contient plus de 97 % en poids d'un ou de plusieurs composés de la formule I.

7. Procédé selon la revendication 3, dans lequel le rapport poids entre l'anhydride d'acide maléique et le solvant n'est pas supérieur à 1:4.

8. Procédé selon la revendication 3, dans lequel le rapport poids entre l'anhydride d'acide maléique et le solvant est de 1:3 à 1:9.

9. Procédé selon la revendication 8, dans lequel le rapport poids entre l'anhydride d'acide maléique et le solvant est de 1:3 à 1:6.

10. Procédé selon la revendication 3, dans lequel la quantité de l'initiateur de type peroxyde est de 4 à 8 % en poids rapportée à l'anhydride d'acide maléique.

11. Procédé selon la revendication 3, dans lequel l'initiateur de type peroxyde est du di-tert-butylperoxyde.

12. Procédé selon la revendication 3, dans lequel l'anhydride obtenu est hydrolysé en acide libre ou en ses sels solubles dans l'eau.

13. Poly(anhydride) d'acide carbonique de formule II

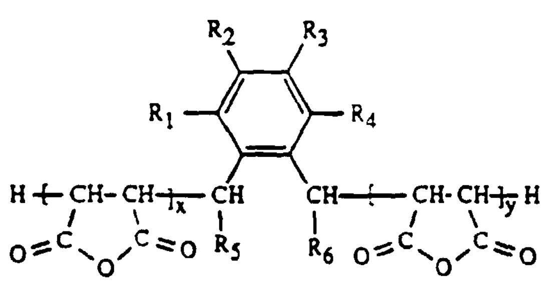

II

dans laquelle $R_1$, $R_2$, $R_3$ et $R_4$ signifient, indépendamment les uns des autres, un atome d'hydrogène, un groupe alkyle $C_1$-$C_4$ ou un groupe carboxyle, $R_5$ et $R_6$ signifient, indépendamment l'un de l'autre, un atome d'hydrogène

ou un groupe méthyle, ou $R_5$ et $R_6$ ensemble signifient un groupe méthylène ou un groupe éthylène, x signifie 1, 2 ou 3 et y 1, 2 ou 3, étant à préciser que x et y ne signifient pas 1 en même temps.

**14.** Anhydride selon la revendication 13, dans lequel $R_1$ à $R_6$ signifient respectivement un atome d'hydrogène ou $R_1$ à $R_3$ $R_5$ et $R_6$ signifient respectivement un atome d'hydrogène et $R_4$ un groupe méthyle, ou $R_1$ signifie un groupe méthyle et $R_2$ a $R_6$ respectivement un atome d'hydrogène, ou $R_1$, $R_4$, $R_5$ et $R_6$ signifient respectivement un atome d'hydrogène et $R_2$ et $R_3$ respectivement un groupe méthyle, ou $R_1$, $R_2$, $R_4$, $R_5$ et $R_6$ signifient respectivement un atome d'hydrogène et $R_3$ un groupe méthyle, ou $R_1$, $R_3$, $R_4$, $R_5$ et $R_6$ signifient respectivement un atome d'hydrogène et $R_2$ un groupe méthyle, ou $R_1$, $R_2$, $R_4$, $R_5$ et $R_6$ signifient respectivement un atome d'hydrogène et $R_3$ un groupe tert-butyle, ou $R_1$ et $R_3$ à $R_6$ signifient respectivement un atome d'hydrogène et $R_2$ un groupe tert-butyle, ou $R_1$, $R_3$, $R_5$ et $R_6$ signifient respectivement un atome d'hydrogène et $R_2$ et $R_4$ respectivement un groupe méthyle, ou $R_2$ et $R_3$ signifient respectivement un groupe méthyle et $R_1$, $R_4$, $R_5$ et $R_6$ respectivement un atome d'hydrogène, ou $R_1$, $R_4$, $R_5$ et $R_6$ signifient respectivement un atome d'hydrogène et $R_2$ et $R_3$ respectivement un groupe carboxyle.

**15.** Anhydride selon la revendication 14, dans lequel x est 1 et y est 2.

**16.** Anhydride selon la revendication 14, dans lequel $R_1$ à $R_6$ sont respectivement des atomes d'hydrogène.

**17.** Mélange contenant au moins un anhydride de la formule II selon la revendication 13 et au moins un anhydride de la formule III

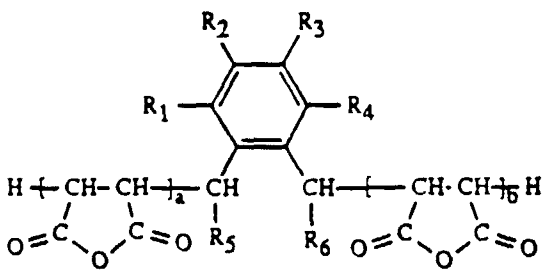

III

dans laquelle $R_1$, $R_2$, $R_3$, $R_4$, $R_5$ et $R_6$ ont la signification indiquée dans la revendication 13, a et b étant respectivement 0 ou 1 et la somme de a+b étant 1 ou 2.

**18.** Mélange contenant au moins un anhydride de la formule II selon la revendication 14 et au moins un anhydride de la formule III

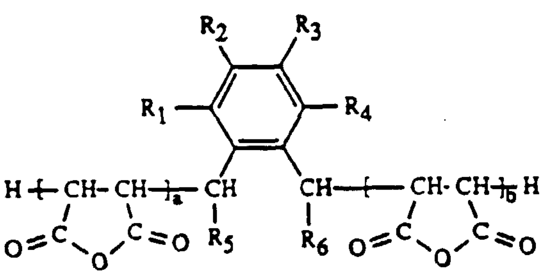

III

dans laquelle $R_1$, $R_2$, $R_3$, $R_4$, $R_5$ et $R_6$ ont la signification indiquée dans la revendication 14, a et b étant respectivement 0 ou 1 et la somme de a+b étant 1 ou 2.

**19.** Mélange selon la revendication 17, contenant au moins un anhydride de la formule II dans laquelle x signifie 1 et y signifie 2.

**20.** Acide polycarbonique ou ses sels solubles dans l'eau, pouvant être obtenu par hydrolyse d'un anhydride selon la revendication 13.

**21.** Acide polycarbonique ou ses sels solubles dans l'eau, pouvant être obtenu par hydrolyse du mélange selon la revendication 17.

**22.** Procédé destiné à l'inhibition de la formation de tartre provenant de l'eau ou d'un système aqueux, caractérisé en ce qu'un poly(anhydride) d'acide maléique selon la revendication 1 est ajouté à l'eau ou aux systèmes aqueux.

**23.** Procédé destiné à l'inhibition de la formation de tartre provenant de l'eau ou d'un système aqueux, caractérisé en ce qu'un poly(anhydride) d'acide carbonique selon la revendication 13, ou un acide polycarbonique ou un sel soluble dans l'eau pouvant être obtenus par hydrolyse de l'anhydride, est ajouté à l'eau ou aux systèmes aqueux.

**Revendications pour l'Etat contractant suivant : ES**

**1.** Procédé destiné à la fabrication de poly(anhydride) d'acide maléique par polymérisation d'anhydride d'acide maléique avec utilisation d'un initiateur de type peroxyde dans une quantité non supérieure à 10 % en poids rapportée à l'anhydride d'acide maléique dans un solvant qui est principalement un o-xylène de la formule I

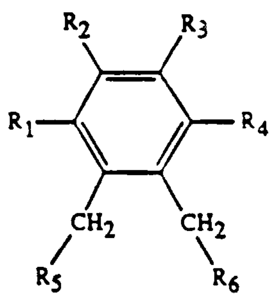

(I)

dans laquelle $R_1$, $R_2$, $R_3$ et $R_4$ signifient, indépendamment les uns des autres, un atome d'hydrogène, un groupe alkyle $C_1$-$C_4$ ou un groupe carboxyle, $R_5$ et $R_6$ signifient, indépendamment l'un de l'autre, un atome d'hydrogène ou un groupe méthyle, ou $R_5$ et $R_6$ ensemble signifient un groupe méthylène ou un groupe éthylène, et dans lequel le rapport des poids entre l'anhydride d'acide maléique et le solvant n'est pas supérieur à 1:3.

**2.** Procédé selon la revendication 1 destiné à la fabrication de poly(anhydride) d'acide maléique dont la moyenne pondérale des poids moléculaires déterminée au moyen de chromatographie par perméation de gel se situe entre 450 et 800 et dont l'indice de polymolécularité se situe entre 1,0 et 1,15.

**3.** Procédé selon la revendication 1 destiné à la fabrication du produit sous forme de polyacide libre ou de son polysel soluble dans l'eau.

**4.** Procédé selon la revendication 1, dans lequel l'o-xylène de la formule I est choisi parmi la série d'un composé dans lequel $R_1$ à $R_6$ signifient un atome d'hydrogène, d'un composé dans lequel $R_2$ à $R_3$, $R_5$ et $R_6$ signifient respectivement un atome d'hydrogène et $R_4$ un groupe méthyle, d'un composé dans lequel $R_2$ signifie un groupe méthyle et $R_2$ à $R_6$ respectivement un atome d'hydrogène, d'un composé dans lequel $R_1$, $R_4$, $R_5$ et $R_6$ signifient respectivement un atome d'hydrogène et $R_2$ et $R_3$ respectivement un groupe méthyle, d'un composé dans lequel $R_1$, $R_2$, $R_4$, $R_5$ et $R_6$ signifient respectivement un atome d'hydrogène et $R_3$ un groupe méthyle, d'un composé dans lequel $R_1$, $R_3$, $R_4$, $R_5$ et $R_6$ signifient respectivement un atome d'hydrogène et $R_2$ un groupe méthyle, d'un composé dans lequel $R_1$, $R_2$, $R_4$, $R_5$ et $R_6$ signifient respectivement un atome d'hydrogène et $R_3$ un groupe tert-butyle, d'un composé dans lequel $R_1$ et $R_3$ à $R_6$ signifient un atome d'hydrogène et $R_2$ un groupe tert-butyle, d'un composé dans lequel $R_1$, $R_3$, $R_5$ et $R_6$ signifient respectivement un atome d'hydrogène et $R_2$ et $R_4$ respectivement un groupe méthyle, d'un composé dans lequel $R_1$ et $R_3$ signifient respectivement un groupe méthyle et $R_2$, $R_4$, $R_5$ et $R_6$ respectivement un atome d'hydrogène, d'un composé dans lequel $R_1$, $R_4$, $R_5$ et $R_6$ signifient respectivement un atome d'hydrogène et $R_2$ et $R_3$ respectivement un groupe carboxyle, et de mélanges de deux ou de plusieurs des composés cités.

5. Procédé selon la revendication 1, dans lequel le solvant contient plus de 95 % en poids d'un ou de plusieurs composés de la formule I.

6. Procédé selon la revendication 5, dans lequel le solvant contient plus de 97 % en poids d'un au de plusieurs composés de la formule I.

7. Procédé selon la revendication 1, dans lequel le rapport poids entre l'anhydride d'acide maléique et le solvant n'est pas supérieur à 1:4.

8. Procédé selon la revendication 1, dans lequel le rapport poids entre l'anhydride d'acide maléique et le solvant est de 1:3 à 1:9.

9. Procédé selon la revendication 8, dans lequel le rapport poids entre l'anhydride d'acide maléique et le solvant est de 1:3 à 1:6.

10. Procédé selon la revendication 1, dans lequel la quantité de l'initiateur de type peroxyde est de 4 à 8 % en poids rapportée à l'anhydride d'acide maléique.

11. Procédé selon la revendication 1, dans lequel l'initiateur de type peroxyde est du di-tert-butylperoxyde.

12. Procédé selon la revendication 1, dans lequel l'anhydride obtenu est hydrolysé en acide libre ou en ses sels solubles dans l'eau.

13. Procédé selon la revendication 1 destiné à la fabrication de poly(anhydride) d'acide carbonique de la formule II

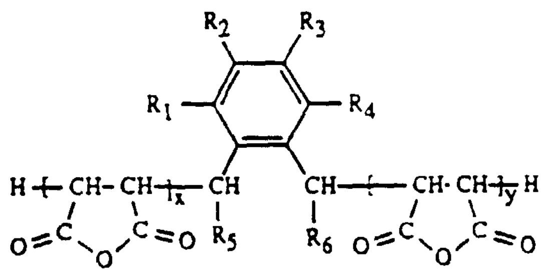

II

dans laquelle $R_1$, $R_2$, $R_3$ et $R_4$ signifient, indépendamment les uns des autres, un atome d'hydrogène, un groupe alkyle $C_1$-$C_4$ ou un groupe carboxyle, $R_5$ et $R_6$ signifient, indépendamment l'un de l'autre, un atome d'hydrogène au un groupe méthyle, ou $R_5$ et $R_6$ ensemble signifient un groupe méthylène ou un groupe éthylène, x signifie 1, 2 ou 3 et y 1, 2 ou 3, étant à préciser que x et y ne signifient pas 1 en même temps.

14. Procédé selon la revendication 13, destiné à la fabrication d'anhydrides, dans lesquels $R_1$ à $R_6$ signifient respectivement un atome d'hydrogène ou $R_1$ à $R_3$, $R_5$ et $R_6$ signifient respectivement un atome d'hydrogène et $R_4$ un groupe méthyle, ou $R_1$ signifie un groupe méthyle et $R_2$ à $R_6$ respectivement un atome d'hydrogène, ou $R_1$, $R_4$, $R_5$ et $R_6$ signifient respectivement un atome d'hydrogène et $R_2$ et $R_3$ respectivement un groupe méthyle, ou $R_1$, $R_2$, $R_4$, $R_5$ et $R_6$ signifient respectivement un atome d'hydrogène et $R_3$ un groupe méthyle. ou $R_1$, $R_3$, $R_4$, $R_5$ et $R_6$ signifient respectivement un atome d'hydrogène et $R_2$ un groupe méthyle, ou $R_1$, $R_2$, $R_4$, $R_5$ et $R_6$ signifient respectivement un atome d'hydrogène et $R_3$ un groupe tert-butyle, ou $R_1$ et $R_3$ à $R_6$ signifient respectivement un atome d'hydrogène et $R_2$ un groupe tert-butyle, ou $R_1$, $R_3$, $R_5$ et $R_6$ signifient respectivement un atome d'hydrogène et $R_2$ et $R_4$ respectivement un groupe méthyle, ou $R_1$ et $R_3$ signifient respectivement un groupe méthyle et $R_2$, $R_4$, $R_5$ et $R_6$ respectivement un atome d'hydrogène, ou $R_1$, $R_4$, $R_5$ et $R_6$ signifient respectivement un atome d'hydrogène et $R_2$ et $R_3$ respectivement un groupe carboxyle.

15. Procédé selon la revendication 14, destiné à la fabrication d'anhydrides, dans lesquels x est 1 et y 2.

16. Procédé selon la revendication 14, destiné à la fabrication d'anhydrides, dans lesquels $R_1$ à $R_6$ sont respectivement des atomes d'hydrogène.

**17.** Mélange contenant au moins un anhydride de la formule II selon la revendication 13 et au moins un anhydride de la formule III

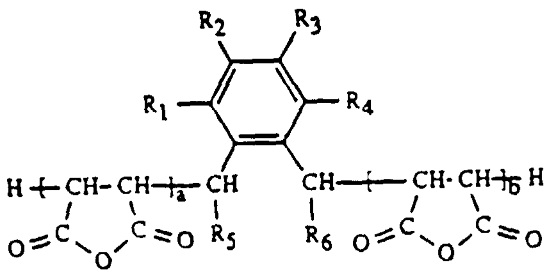

III

dans laquelle $R_1$, $R_2$, $R_3$, $R_4$, $R_5$ et $R_6$ ont la signification indiquée dans la revendication 13, a et b étant respectivement 0 ou 1 et la somme de a+b étant 1 ou 2.

**18.** Mélange contenant au moins un anhydride de la formule II selon la revendication 14 et au moins un anhydride de la formule III

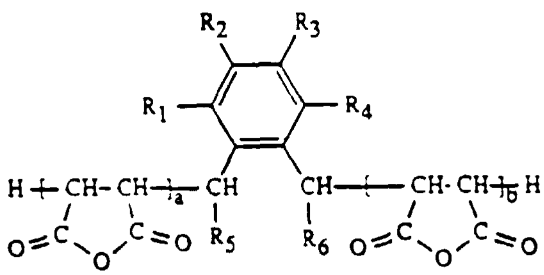

III

dans laquelle $R_1$, $R_2$, $R_3$, $R_4$, $R_5$ et $R_6$ ont la signification indiquée dans la revendication 14, a et b étant respectivement 0 ou 1 et la somme de a+b étant 1 ou 2.

**19.** Mélange selon la revendication 17, contenant au moins un anhydride de la formule II dans laquelle x signifie 1 et y signifie 2.

**20.** Procédé destiné à la fabrication d'acides polycarboniques ou de leurs sels solubles dans l'eau par hydrolyse d'un anhydride selon la revendication 13.

**21.** Procédé destiné à la fabrication d'acides polycarboniques ou de leurs sels solubles dans l'eau par hydrolyse du mélange selon la revendication 17.

**22.** Procédé destiné à l'inhibition de la formation de tartre provenant de l'eau ou d'un système aqueux, caractérisé en ce qu'un poly(anhydride) d'acide maléique selon la revendication 1 est ajouté à l'eau ou aux systèmes aqueux.

**23.** Procédé destiné à l'inhibition de la formation de tartre provenant de l'eau ou d'un système aqueux, caractérisé en ce qu'un poly(anhydride) d'acide carbonique selon la revendication 13, au un acide polycarbonique ou un sel soluble dans l'eau pouvant être obtenu par hydrolyse de l'anhydride, est ajouté à l'eau ou aux systèmes aqueux.